# EUROPEAN PATENT SPECIFICATION

(11) **EP 3 075 260 B1**
(45) Date of publication and mention of the grant of the patent: **11.09.2019**
(21) Application number: 14864455.2
(22) Date of filing: 21.11.2014
(51) Int. Cl.: A23K 10/40, B01J 20/18, C07C 217/44, B01J 20/12, C01B 39/02, B01J 39/02, A61K 9/00, A61K 9/14, B01J 39/14, B01J 20/22, B01J 20/32

(54) **MYCOTOXIN ADSORBENT AND THE USE THEREOF IN BALANCED FOOD FOR ANIMALS**
MYKOTOXINADSORPTIONSMITTEL UND VERWENDUNG DAVON IN AUSGEGLICHENEN NAHRUNGSMITTELN FÜR TIERE
ADSORBANT DE MYCOTOXINES ET SON UTILISATION DANS DES ALIMENTS ÉQUILIBRÉS POUR ANIMAUX

(30) Priority: 25.11.2013 MX 2013013788
(43) Date of publication of application: 05.10.2016
(73) Proprietor: NUTEK, S.A. DE C.V., 75700 Tehuacán, Puebla (MX)
(72) Inventor: LARA ARELLANO, Javier Armando, 75760 Tehuacán, Puebla (MX); ROMERO MARTÍNEZ DEL SOBRAL, Miguel Ángel, 75700 Tehuacán, Puebla (MX); GARCÍA ROSAS, Irlanda Verónica, 75700 Tehuacán, Puebla (MX); FIERRO HUESCA, José Antonio, 75740 Tehuacán, Puebla (MX)
(74) Representative: Padial Martinez, Ana Belen
(86) International application number: PCT/IB2014/066246
(87) International publication number: WO 2015/075686

(56) References cited:
- WO-A1-00/41806
- WO-A1-02/07875
- WO-A1-02/052950
- WO-A1-02/064502
- SANG-MO KOH ET AL: "Preparation and application of organo-minerals as sorbents of phenol, benzene and toluene", APPLIED CLAY SCIENCE, vol. 18, no. 3-4, 14 February 2001 (2001-02-14), pages 111-122, XP055387039, AMSTERDAM, NL ISSN: 0169-1317, DOI: 10.1016/S0169-1317(00)00040-5
- DULTZ STEFAN ET AL: "Organic cation exchanged montmorillonite and vermiculite as adsorbents for Cr(VI): Effect of layer charge on adsorption properties", APPLIED CLAY SCIENCE, vol. 67, 12 July 2012 (2012-07-12), pages 125-133, XP028958260, ISSN: 0169-1317, DOI: 10.1016/J.CLAY.2012.05.004
- TOMASEVIC-CANOVIC M ET AL: "Surfactant modified zeolites--new efficient adsorbents for mycotoxins", MICROPOROUS AND MESOPOROUS MATER, ELSEVIER, AMSTERDAM, NL, vol. 61, no. 1-3, 18 July 2003 (2003-07-18), pages 173-180, XP004437471, ISSN: 1387-1811, DOI: 10.1016/S1387-1811(03)00365-2
- S L LEMKE ET AL.: 'Adsortion of zearalenone by organophilic montmorillonite clay' JOURNAL AGRICULTURE FOOD CHEMISTRY 1998, pages 3789 - 3796, XP000940529

## Description

### TECHNICAL FIELD OF THE INVENTION

The present invention refers to a mycotoxin adsorbent used in balanced feeds for preventing adverse effects of type A and B trichothecenes and with a special emphasis on vomitoxin (or deoxynivalenol) and T-2 toxin in animals. Furthermore, this invention also refers to an additive for balanced animal feed or formulation thereof that includes said mycotoxin adsorbent; and the said mycotoxin for use in the treatment or prevention of one or more adverse effects or digestive tract symptoms associated with trichothecene intoxication, particularly type A and B trichothecenes, and particularly vomitoxin (or deoxynivalenol) and T-2 toxin.

### BACKGROUND

Mycotoxins are chemical compounds of low molecular weight, produced by mushrooms that produce pathological effects in humans as well as in animals. There are hundreds of mycotoxins that are produced by many different kinds of mushrooms which contaminate grains and animal food, whether in the fields or in grain storage silos. In the fields, the mushroom that most often affects grains is the *Fusarium sp* which produces the toxins zearalenone, fumonisin and trichothecenes (vomitoxin, T-2 toxin, DAS), among others.

In particular, trichothecenes are mycotoxins mainly found in various species of mushrooms of the genus *Fusarium* (e.g., *F. Sporotrichioides, F. graminearum, F. poae* and *F. culmorum*) and can be found in members of other genuses as well, such as: *Myrothecium, Cephalsporium, Trichoderma* and *Trichothecium.* Trichothecenes are chemically characterized by the presence of a basic system of a tetracycline scirpenol ring. Chemically, trichothecenes are compounds that have sesquiterpenes rings characterized by a nucleus of 12,13-epoxy-9-trichothecene and they have a variable number of substitutions with hydroxyl or acetoxy groups in the 3, 4, 7, 8 and 15 molecular positions. Commonly, some trichothecenes are only differentiated by a single acetyl group.

In all, there are four groups of trichothecenes. However, only the Fusarium genus produces type A and B trichothecenes. Considering that for economic reasons the focus is on trichothecenes of the Fusarium genus, these two groups are of primary interest. Examples of type A trichothecenes are T-2 and HT2 toxins, diacetoxyscirpenol (DAS) and neosalaniol, and among the most important type B trichothecenes are deoxinivalenol (DON), more widely known as vomitoxin, and nivalenol.

The basic structure of trichothecenes is shown in formula (I) and the substitutions for this basic structure that forms groups A and B are listed in Table 1. 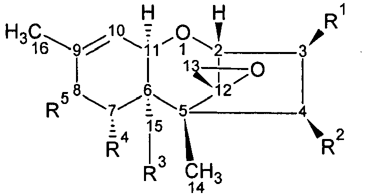 **Formula (I).** Basic trichothecene structure.

**Table 1. Trichothecene structure in groups A and B.**

| Trichothecenes | PM^{a} | R¹ | R² | R³ | R⁴ | R⁵ |
|---|---|---|---|---|---|---|
| **Type A** | | | | | | |
| Diacetoxiscirpenol (DAS) | 366 | OH | OAc | OAc | H | H |
| HT-2 (HT-2) toxin | 424 | OH | OH | OAc | H | OCOCH2CH(CH3)2 |
| T-2 (T-2)toxin | 466 | OH | OAc | OAc | H | OCOCH2CH(CH3)2 |
| Neosolaniol (NEO) | 382 | OH | OAc | OAc | H | OH |

| **Type B** | | | | | | |
|---|---|---|---|---|---|---|
| Deoxinivalenol (DON) | 296 | OH | H | OH | OH | O |
| 3-acetyl Deoxinivalenol (3-AcDON) | 338 | OAc | H | OH | OH | O |
| Nivalenol (NIV) | 312 | OH | OH | OH | OH | O |
| Fusarenone-X (Fus-X) | 354 | OH | OAc | OH | OH | O |
| Tricotecolone (TRI) | 264 | H | OH | H | H | O |

| | | | | | | |
|---|---|---|---|---|---|---|
| ^{a} Molecular weight (g/mol), R1-R5 are expressed in Formula 1. | | | | | | |

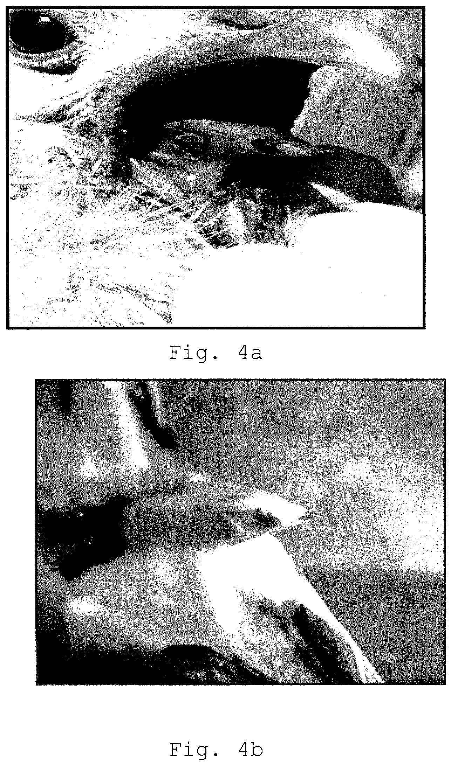

Commonly, trichothecenes produce the following adverse effects in animals: vomiting, diarrhea, irritation, hemorrhaging and necrosis in the digestive tract. Particularly, mycotoxins in this group are the T-2 toxin and diacetoxiscirpenol (DAS); oral lesions caused by these mycotoxins in birds are characteristic (see Figure 4). In contrast, the most prevalent trichothecene in grains and balanced feed most commonly found in high levels of concentration is deoxinivalenol (DON) or vomitoxin, the latter which was named according to the effect of vomiting it produces, and it is also known to cause animals to reject their feed.

It has been observed that the presence of trichothecenes in balanced animal feed reduces their consumption of the feed. Particularly pigs can be very sensitive to the effects of trichothecenes.

Although the difference between the toxic effects of T-2 toxin and vomitoxin (DON) has not been widely reported in respect to feed consumption, both toxins differ notably in terms of their other toxic effects. In both cases, the effects of these toxins are due to the alterations they cause in dopamine, tryptophan, serotonin, and serotonin metabolite levels in the brains of rodents and pigs (*Prelusky et al 1992*).

The change in the concentration of these substances in the brain has been observed to be similar to that which is produced by anorexic substances, indicating that the reduction of the animal feed consumption is at least in part due to the alteration of neurotransmitter levels in the brain. It is likely that in the future a relationship will be found between these toxics effects and the peripheral nervous system.

In consideration of the foregoing, the industry is convinced that the best way to deal with the problem of mycotoxins in animal feeds is prevention. In so doing, efforts have been made to implement adequate grain management programs to reduce the likelihood the grain and the feed being contaminated with these toxins. This includes attempting to implement programs to prevent already contaminated grains from even reaching the storage tanks.

Thus, investigations have been underway of various methods of treating the contaminated grain to obtain good quality grains. However, all these methods have been insufficient in preventing contamination. In this respect, according to the United Nations Food and Agriculture Organization (FAO), a large part of the world's grains are contaminated with mycotoxins. The reason for this assessment is precisely due to the failure of such programs implemented in the industry until now (*Bhat and Vasanthi 1999*).

One of the proposed solutions for solving the problem of mycotoxin contamination in animal feed is to use mycotoxin adsorbents, which are utilized as additives in feed. They function by trapping and adsorbing the mycotoxins when they are in the aqueous environment of the gastrointestinal tract of the animal once it consumes the contaminated feed. These mycotoxin adsorbents essentially prevent mycotoxins from being absorbed by the animal and reaching its circulatory system, where they can cause their adverse effects. The use of aluminum silicates, clays, zeolites, including organic aluminum silicates as mycotoxin adsorbents (*Phillips et al 1988, Kubena et al 1990*) has been a widely known practice.

In prior art, several documents exist that refer to mycotoxin adsorbents. For example, the document DE3810004 (1989) discloses the use of bentonite for binding mycotoxins in humans and animals. This document reveals that bentonite is effective for binding zearalenone, some trichothecenes (such as deoxinivalenol and T-2 toxin), ocratoxine and PR toxin.

In addition, the document WO9113555 (1991) describes a solid, dry, biodegradable composition, recovered by a sequestering agent, to be used as an additive for adsorbing mycotoxins in contaminated feed. Said composition comprises a phyllosilicate mineral such as calcium montmorillonite.

The S.L. Lemke, P.G. Grant and T.D. Phillips document, "Adsorption of Zearalenone by Organophilic Montmorillonite Clay" J Agric. Food Chem. (1998), pp. 3789-3796, describes a montmorillonite clay or an organically modified (organophyllic) montmorillonite clay that is capable of adsorbing zearalenone.

The document WO 00/41806 (2000) discloses mycotoxin adsorbents that comprise an organically modified layered silicate that comprises a quaternary onium compound, wherein said onium compound includes at least one alkyl group C10-C22 and an aromatic substituent, and wherein 2 of 30% of the exchangeable cations of layered silicate are exchanged for quaternary onium compounds.

In addition, we found in the prior art the document WO02052950 (2002), which describes an organic mineral modified with a long-chain quaternary amine, for instance, with dioctadecyltrimethylamine, octadecyltrimethylamine, octadecyldimethylamine and similar compounds. This modified organic mineral is used as an additive in feed for adsorbing mycotoxins in animals.

The document US20040028678 (2004) describes the use of a layered silicate activated with acid for adsorbing aflatoxins, ocratoxins, fumonisine, zearalenone, deoxinivalenol, T-2 toxin and ergotamine.

The document US20080248155 (2008) describes the use of a composition that comprises stevensite for adsorbing mycotoxins, such as for example the T-2 mycotoxin.

The document US20100330235 (2010) describes an adsorbent of mycotoxins such as aflatoxin, zearalenone, ocratoxin A and fumonisine B1, based on the combination of an organic silicate with an amorphic and dodecylamine structure (a primary amine that has a carbon chain of twelve aliphatic, linear and non-polar carbons).

Recently, other solutions have also been proposed to solve the problem of decontaminating feed with mycotoxins. For example, the document US20120070516 (2012) discloses a method for making feed contaminated with mycotoxins such as biomass; We also found the document US20120219683 (2012) which describes an adsorbent of mycotoxins such as deoxinivalenol and T-2 toxin that comprises a clay material and activated carbon for decontaminating undesirable mycotoxins in feed.

Song-Mo Koh et al. "Preparation and application of organo-minerals as sorbents of phenol, benzene and toluene", APPLIED CLAY SCIENCE, vol. 18, No 3-4, pp. 11-122 (February 14, 2001) discloses a material comprising montmorillonite with a cation exchange capacity of 103 meq/100 g or clinoptilolite-zolite 27 meq/100 g, and benzethonium chloride.

Dultz, Stefan et al. "Organic cation exchanged montmorillonite and vermaculite as adsorbents for Cr (VI): Effect of layer charge on adsorption properties", APPLIED CLAY SCIENCE, vol. 67, pp. 125-133 (July 12, 2012) discloses another absorbent comprising Wyoming bentonite with a cation exchange capacity of 76 meq/100 g or Russian vermiculite with a cation exchange capacity of 162 meq/100 g, and benzethonium chloride., WO 02/064502 A1 discloses an organozeolite absorbent for mycotoxins comprising layers of quaternary ammonium compounds such as octadecylbenzyldimethyl and a zeolite tuff.

Thomasevic-Canovic M. et al "Surfactant modified zeolite - new efficient absorbents for mycotoxins", MICROPOROUS AND MESOPOROUS MATTER, vol. 61, No 1-3, pp 173-18, ELSEVIER, AMSTERDAM (July 18, 2003) discloses another organozeolite absorbent for mycotoxins comprising layers of quaternary ammonium compounds such as octadecylbenzyldimethyl ammonium chloride and dioctadecylbenzyldimethyl ammonium chloride and a zeolite tuff.

Nevertheless, none of the mycotoxin adsorbents described in the previous art have the ability to reduce the bioavailability of trichothecenes. In the case of T-2 toxin, the reduction of its bioavailibity has been limited and in the case of vomitoxin, none.

In the present application, we maintain that the lack of effective adsorption of vomitoxin (DON) and/or T-2 toxin in the known mycotoxin adsorbents is mainly due to the highly polarized characteristics of said toxins, resulting in a much greater interaction between vomitoxin (DON) and/or T-2 toxin in aqueous environments than with the surface of the known mycotoxin adsorbents.

This proposal will also help explain the reason why known organic aluminum silicates that comprise a non-polar alkyl chain and that have been used until now to adsorb mycotoxins known mycotoxin adsorbents.

This proposal will also help explain the reason why known organic aluminum silicates that comprise a non-polar alkyl chain and that have been used until now to adsorb mycotoxins other than aflatoxins have proven to not be very effective for the adsorption of vomitoxin. Therefore, we did not find in the prior art any documentation of a comprehensive solution to the problem of decontaminating feed with mycotoxins, particularly in preventing, reducing, or eliminating the adverse effects or symptoms in the digestive tract of animals associated with intoxication by trichothecenes, especially A and B trichothecenes, and even more specifically, the toxic effects associated with intoxication by vomitoxin and T-2 toxin.

### BRIEF DESCRIPTION OF THE INVENTION

The inventors of the present application have unexpectedly found a mycotoxin adsorbent that allows high adsorption of vomitoxin as well as the T-2 toxin, whose particular vomitoxin adsorption levels have been found to be particularly high, and while maintaining high adsorption rates of other mycotoxins, such as aflatoxins, ocratxin A, fumonisine B1 and zearalenone.

In a first aspect of the present application, the invention refer to a mycotoxin adsorbent according to claim 1.

The invention additionally refers to the mycotoxin adsorbent for use in the treatment or prevention of one or more adverse effects or symptoms in the digestive tract associated with intoxication by Type A and/or B trichothecenes, and especially vomitoxin (or deoxinivalenol) and T-2 toxin, according to claim 7. The present invention further provides a process for preparing a mycotoxin adsorbent according to claim 8.

Thus, carrying out the present invention prevents the absorption of mycotoxins in contaminated feed by the gastrointestinal tract of the animal, thereby substantially improving the health of the animals, reflected in animal weight gain as well as in the productivity of derived products like eggs and milk.

### BRIEF DESCRIPTION OF THE FIGURES

Figure 1 provides a graphical representation of the results of body weight after 23 days of experimenting with the mycotoxin adsorbent of the invention to combat DON in pigs (the different letters statistically vary by p<0.05)
Figure 2 provides a graphical representation of weight gain after 23 days of experimenting with the use of the mycotoxin adsorbent of the invention to combat DON in pigs (the different letters statistically vary by p<0.05)
Figure 3 provides a graphical representation of body weight after 28 days of experimenting *in vivo* using the mycotoxin adsorbent of the invention to combat T-2 toxin in chickens (the different letters statistically vary by p<0.05)
Figures 4A and 4B show oral lesions due to the effect of T-2 on broiler chickens.
Figure 5 provides a graphical representation of body weight after 23 days of testing the use of the mycotoxin adsorbent of the invention to combat DON in pigs (the different letters statistically vary by p<0.05)
Figure 6 provides a graphical representation of weight gain after 23 days of experimenting with the use of the mycotoxin adsorbent of the invention to combat DON in pigs (the different letters statistically vary by p<0.05)

### DETAILED DESCRIPTION

The fact that the surface of aluminum silicates can be treated for modification in such a way as to increase its adsorption capacity of mycotoxins is the fundamental premise of the present invention. The treatment to follow depends on the objective to be achieved, but generally, the treatment involves manipulating two main characteristics of the surface: its hydrophobic and its organophyllic properties, respectively (*Lara et al 1998*).

Particularly, the surface in the present invention is modified by a functionalized-chain organic compound whereby the surface attains a highly polar quality. The organic compound that is used to modify the Surface can occupy a portion or all of the active sites of the aluminum silicate surface.

The aluminum silicate used can be a tectosilicate or a phyllosilicate, or a mixture of both, under the condition that the material used has a cation exchange capacity of least 20 milliequivalents per 100 grams of material, and preferably 55 milliequivalents per 100 grams of material.

The selection of the organic compound depends on the specificity and efficiency desired in the mycotoxin adsorption, but in general, the organic compound used is a derivative of alkyl phenol ethoxylate. This organic compound is used in a proportion of 25% to 120% of the cation exchange capacity of the aluminum silicate used. The reaction takes place in an aqueous environment by agitating it at a temperature between 15 and 85°C for 0.25 to 3 hours. The product is separated by filtration, and is dried at a temperature between 40 and 150 °C and is crushed and ground at a temperature between 100 and 325°C.

The additive subject matter of the present invention is an adsorbent of low inclusion which is added to feeds contaminated with trichothecenes, at a rate of 0.025% to 0.2% of feed weight.

The invention refers to a mycotoxin adsorbent comprising an aluminum silicate organically modified with an alkyl phenol ethoxylate derivative with the formula (I): 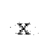 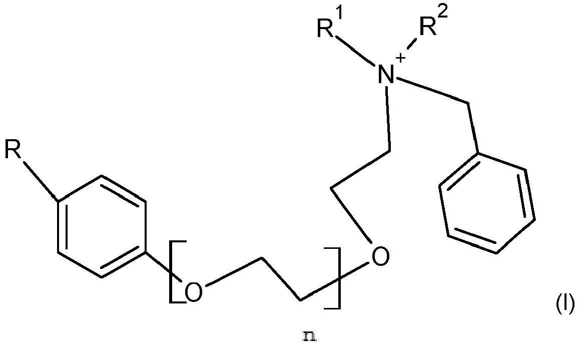 wherein R is n-nonyl, X is bromine and n is 9, and wherein R¹ is methyl and R² is methyl, and wherein the aluminium silicate has a cation exchange capacity of at least 20 milliequivalents per 100 g of material.

In another embodiment, the invention refers to a mycotoxin adsorbent comprising an aluminum silicate organically modified with an alkyl phenol ethoxylate with the formula (I), whereby the aluminum silicate can be a tectosilicate, a phyllosilicate, or a combination of both.

In an embodiment, the mycotoxin adsorbent of the invention, comprises an aluminum silicate organically modified with an alkyl phenol ethoxylate derivative that has formula (I) and that has a cation exchange capacity of at least 55 milliequivalents per 100 grams of material. The mycotoxin adsorbent of the invention, can also comprise the aluminum silicate organically modified with an alkyl phenol ethoxylate derivative that has the formula (I), that is present in a proportion of 25% to 120% of the cation exchange capacity of the aluminum silicate.

The invention further refers to an additive for balanced animal feed comprising the mycotoxin adsorbent.

In addition, the invention refers to a formulation of balanced animal feed that includes a mycotoxin adsorbent in accordance with the invention, as well as the mycotoxin adsorbent of the invention for use in the treatment or prevention of one or more adverse effects or symptoms in the digestive tract associated with type A and/or B trichothecene intoxication.

The invention involves the use of a mycotoxin adsorbent, comprising an aluminum silicate organically modified with an alkyl phenol ethoxylate derivative that has the formula (I) or (Ia) in the preparation of an additive for balanced animal feed for the treatment or prevention of one or more adverse effects or symptoms in the digestive tract associated with trichothecene intoxication of types A and/or B, such as vomiting, diarrhea, irritation, hemorrhaging, or necrosis in the digestive tract associated with trichothecene intoxication. In the present invention, one or more adverse effects or one or more symptoms in the digestive tract associated with trichothecene intoxication are attributed to the group which includes: diacetoxiscirpenol (DAS), HT-2 (HT-2) toxin, T-2 (T-2) toxin, neosolaniol (NEO), deoxinivalenol (DON) or vomitoxin, 3-acetyldeoxinivalenol (3-AcDON), nivalenol (NIV), fusarenone-X (Fus-X), tricotecolone (TRI) or any combination thereof.

The invention also consists of a mycotoxin adsorbent in accordance with the invention for use as an additive for animal fodder for the treatment and prevention of one or more adverse effects or one or more symptoms in the digestive tract associated with trichothecene intoxication, whereby the animal fodder additive is for the treatment or prevention of vomiting, diarrhea, irritation, hemorrhaging, necrosis or oral lesions.

The present invention additionally refers to a process for preparation of a mycotoxin adsorbent comprising the following steps:
a) contacting an aluminum silicate with cation exchange capacity of at least 20 milliequivalents per 100 grams of material with an alkyl phenol ethoxylate derivative that has the formula (I) in an aqueous medium agitating it at a temperature between 15°C and 85°C and for 0.25 to 3 hours at a proportion of 25% to 120% of the cation exchange capacity of the aluminum silicate;
b) Separate by filtration;
c) Dry at a temperature between 40°C and 150°C; and
d) Crush and grind with mesh between 100 and 325.

The following examples show that the objective value of adsorption of vomitoxin and of T-2 toxin when applying the mycotoxin adsorbent of the invention is particularly high. Said examples that include alternatives for obtaining the organic compound derivative of alkyl phenol ethoxylate, the preparation of the mycotoxin adsorbent and its "live" evaluation are provided as a means of illustration rather than limitation.

### EXAMPLE 1

### METHOD OF SYNTHESIZING AN ALKYL PHENOL ETHOXYLATE DERIVATIVE. (METHOD USING TRIPHENYLPHOSPHINE)

### RAW MATERIALS TO YIELD 49 G.

Triphenylphosphine, 60 g
Triethylamine 21 mL
Methylene chloride 300 mL
Bromine 12 mL
Nonyl phenol ethoxylate 10 moles of ethylene oxide 50 g dissolved in 50 mL of methylene chloride.
Hexane-ethyl acetate 1:1
Ethyl ether
Hexane
Ethyl acetate.
Benzyldimethylamine 10.7 mL

### PROCEDURE

1. In a 1000-mL round-bottomed beaker equipped with a magnetic agitator, add 300 mL of methylene chloride and dissolve 60 g of triphenylphosphine and 21 mL triethylamine.
2. Chill the mixture at 0 ºC in an ice bath.
3. Using an open system dropping funnel, slowly add 12 mL of bromine.
4. Once the bromine has been added, allow mixture to react at the same temperature at constant agitation for 10 minutes
5. Dissolve 50g of surfacpol 9010 in 50 ml of methylene chloride and add to reaction beaker.
6. Allow to react at 0 ºC for an additional 30 minutes.
7. Afterward, remove from ice bath and allow reaction to reach room temperature by agitating it for an additional 2 hours.
8. Afterward, filter the reaction mixture in a Büchner funnel with a porous glass bottom sealed with silica.
9. Evaporate the solvent from the strain at low pressure, dissolve the residue in a mixture of hexane-ethyl acetate at 1:1 producing a precipitate (triphenylphosphine oxide) which is eliminated in the filtration process.
10. While the triphenylphosphine oxide precipitate is being produced, wash the residue with the hexane-ethyl acetate mixture again.
11. Dilute the yellow oil in ethyl ether and add hexane to crystalize the secondary residues, and finally, filter the liquid one more time using a Büchner funnel with porous glass bottom sealed with silica.
12. Concentrate the strain at reduced pressure, obtaining 47 g of a slightly yellow oil, which can immediately be used for the second phase of the reaction without prior purification.
13. Mix 47 g of Surfacpol 9010 bromide with 10.7 ml of benzyldimethylamine in a round 250-mL beaker and agitate.
14. Heat the mixture at 120°C for 24 hours, then allow to cool at room temperature.
15. Add sufficient methylene chloride and purify the crude mixture in a column of Flash silica gel, first eluting it with methylene/methanol chloride 20:1, gradually increasing the polarity with anhydrous methanol until obtaining an eluent mixture of CH₂Cl₂/MeOH 2:1.
16. This produces 49 g of an alkyl phenol ethoxylate derivative of the invention (hereinafter: "QUAT Q5" or "Q5"), a Brown oil and with a 75% extraction efficiency by the two phases.

### EXAMPLE 2

### METHOD OF SYNTHESIZING AN ALKYL PHENOL ETHOXYLATE DERIVATIVE. (METHOD USING TRIBROMIDE PHOSPHOROUS)

### RAW MATERIALS TO YIELD 38 G.

Nonyl phenol ethoxylate 10 moles ethylene oxide 50 g
Chloroform 100 mL
Tribromide phosphorous PBr₃ 4 mL.
Dimethylbenzilamine 10 mL
Solvents for elution, dichloromethane and methanol.

### PROCEDURE

1. In a round-bottomed 500-mL beaker dissolve 50 g of the nonyl phenol ethoxylate 10 in 150 mL of chloroform, bring solution to 0 °C in an ice-bath, and agitate for 15 minutes.
2. Add 4 mL of PBr₃ using a syringe, agitate the mixture at the same temperature for 1 hour.
3. Agitate, maintaining at room temperature for 48 h.
4. Evaporate the solvent at reduced pressure.
5. Purify the crude mixture using chromatography in a column using the CH₂Cl₂/MeOH 15:1 system as eluent.
6. Transfer the resulting product to a round-bottomed 250 mL beaker and add 10 ml dimethylbenzilamine.
7. First, heat the mixture at 120°C for 24 hours, then chill the mixture at room temperature.
8. Purify the product by columns using the CH₂Cl₂/MeOH 15:1 system as eluent, yielding 38 grams of alkyl phenol ethoxylate derivative of the invention (50% extraction efficiency), hereinafter: "QUAT Q5" or "Q5".

### EXAMPLE 3

### PREPARATION OF THE MYCOTOXIN ADSORBENT OF THE INVENTION

### RAW MATERIALS TO BE USED.

- The characteristics of the organic compound used to treat the surface as shown in Table 2.
- The aluminum silicate base used is an aluminum silicate of the bentonite type, with a cation exchange capacity of 55 meq/100g.

**Table 2. Characteristics of the alkyl phenol ethoxylate derivative "QUAT 5" or "Q5".**

| | | | | |
|---|---|---|---|---|
| QUATERNARY | TYPE | STRUCTURE | MOLECULAR WEIGHT | CHARGE |

- Grind the sample.
- Strain the simple in a sieve mesh 200.
- Analyze the samples accordingly.

### EXAMPLE 4

### Live testing of the mycotoxin adsorbent of the invention designed for adsorption of vomitoxin (or deoxinivalenol) on pigs.

An aluminum silicate organically modified with an alkyl phenol ethoxylate derivative that has the formula (I), referred to as "QUAT 5" or "Q5," is used as mycotoxin adsorbent in this example.

### Analysis of the results from Example 4

18 recently weaned female piglets were used in this experiment. They were distributed in 3 groups, 6 pigs in each group, each animal considered a replicate. Their diets were identified as shown in Table 4.

**Table 4. Experimental design for the live testing of mycotoxin adsorbent of the invention to combat DON.**

| GROUP | DON ppm | ADSORBENT | DOSE AD kg/T | PIGS/REPLICATE | REPLICATES | TOTAL PIGS |
|---|---|---|---|---|---|---|
| 1 | 0 | NO | 0 | 1 | 6 | 6 |
| 2 | 4.5 | NO | 0 | 1 | 6 | 6 |
| 3 | 4.5 | Q5 | 1.5 | 1 | 6 | 6 |
| | | | | | TOTAL | 18 |

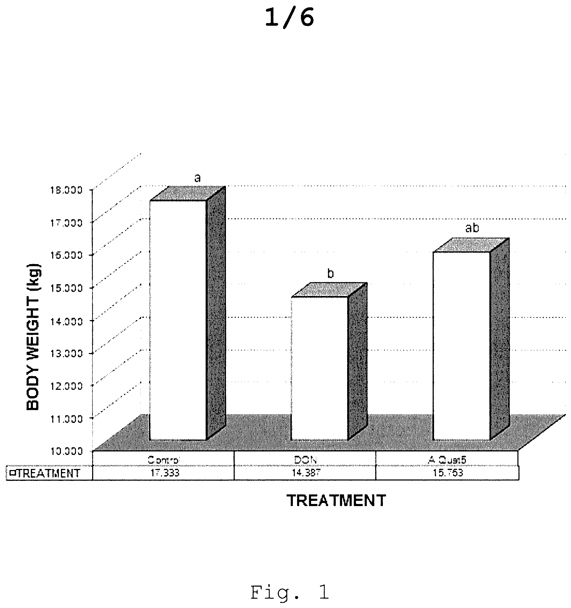

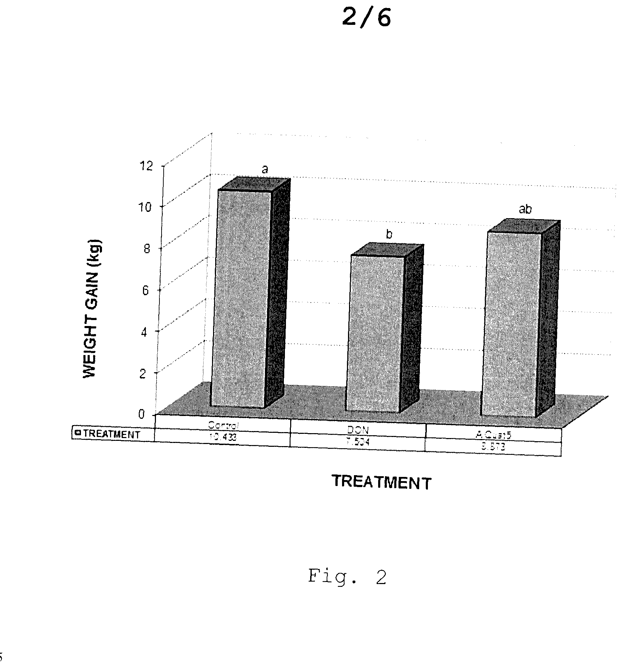

The body weight results following 23 days of testing are graphically represented in Figure 1 for the final weights and in Figure 2 for accumulated weight gain.

Figures 1 and 2 additionally clearly demonstrate that the "QUAT 5" or "Q5" product protected the animals from vomitoxin intoxication. The effect of the toxin was observed in the group that only consumed vomitoxin, thus exhibiting statistical differentiation from the negative control group. The efficacy of the "QUAT 5" or "Q5" product can be calculated with respect to the recuperation of weight as compared to the negative control group. Therefore, it can be concluded that the experimental product "QUAT 5" or "Q5" protected the animal at a rate of 47.7%.

### Analysis of the results from Example 4

18 recently weaned female piglets were used in this experiment. They were distributed in 3 groups, 6 pigs in each group, each animal considered a replicate. Their diets were identified as shown in Table 4.

**Table 4. Experimental design for the live testing of mycotoxin adsorbent of the invention to combat DON.**

| GROUP | DON ppm | ADSORBENT | DOSE AD kg/T | PIGS/REPLICATE | REPLICATES | TOTAL PIGS |
|---|---|---|---|---|---|---|
| 1 | 0 | NO | 0 | 1 | 6 | 6 |
| 2 | 4.5 | NO | 0 | 1 | 6 | 6 |
| 3 | 4.5 | Q5 | 1.5 | 1 | 6 | 6 |
| | | | | | TOTAL | 18 |

The body weight results following 23 days of testing are graphically represented in Figure 1 for the final weights and in Figure 2 for accumulated weight gain.

Figures 1 and 2 additionally clearly demonstrate that the "QUAT 5" or "Q5" product protected the animals from vomitoxin intoxication. The effect of the toxin was observed in the group that only consumed vomitoxin, thus exhibiting statistical differentiation from the negative control group. The efficacy of the "QUAT 5" or "Q5" product can be calculated with respect to the recuperation of weight as compared to the negative control group. Therefore, it can be concluded that the experimental product "QUAT 5" or "Q5" protected the animal at a rate of 47.7%.

### Conclusion of Example 4

Based on the results, it can be concluded that the mycotoxin adsorbent "QUAT 5" or "Q5" is a product that helps to reduce or eliminate the adverse effects or symptoms in the digestive tract associated with trichothecene intoxication in animals, particularly the adverse effects of type A and/or B trichothecenes, and even more specifically, of deoxinivalenol (or vomitoxin).

### EXAMPLE 5

### Live testing of mycotoxin adsorbent of the invention to combat the adverse effects of 1.8 ppm of T-2 toxins obtained from a culture of Fusarium Sporotrichioides, in young broiler chickens (1 to 28 days old).

In this example, a mycotoxin adsorbent which that comprises an organic aluminum silicate prepared with a quaternary of ammonium derived from the alkyl phenol ethoxylate with the formula (I) was tested. Said mycotoxin adsorbent referred to in the foregoing as "QUAT 5" or "Q5". We also evaluated another mycotoxin adsorbent, which comprises an organic aluminum silicate prepared with a quaternary of ammonium with a high-polarity chain derived from glucose, hereinafter referred to as "QUAT 3" or "Q3".

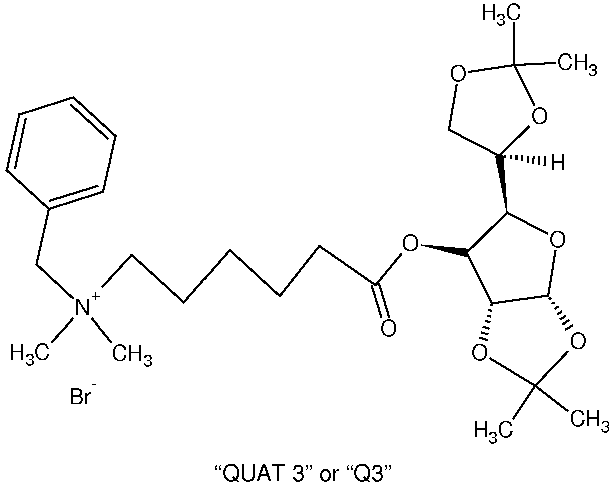

The T2 toxin used in this example is obtained from a *Fusarium Sporotrichioides* culture and it was combined with industrial feed for broiler chickens.

112 1-day-old chickens were used in the present trial, they were distributed in 4 treatments of 4 repetitions with 7 chickens per replicate. Table 5 shows the distribution of the treatments.

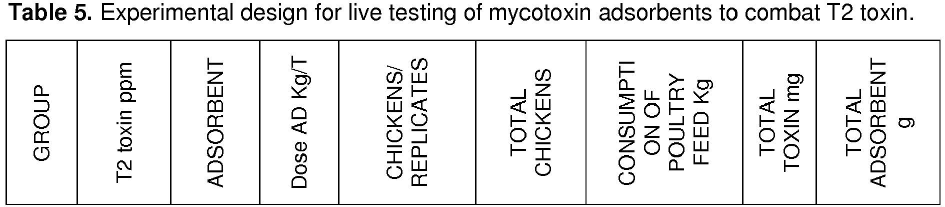

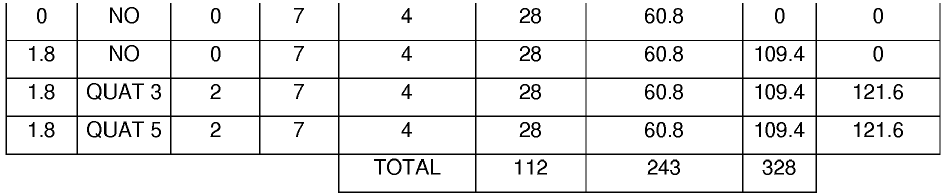

### Response variables to be determined

a. Productive or performance variables.
   - Weekly body weight
   - Weekly and total consumption of feed.
   - Weekly and cyclical feed conversion.
b. Toxic-pathological variables.
   - Mortality.
   - Observation of oral lesions.

At the end of the experiment, 8 chickens were sacrificed per treatment, i.e., 2 per replicate for relative weight of the following organs: kidneys, liver and bursa of Fabricius.

### Analysis of the results of Example 5

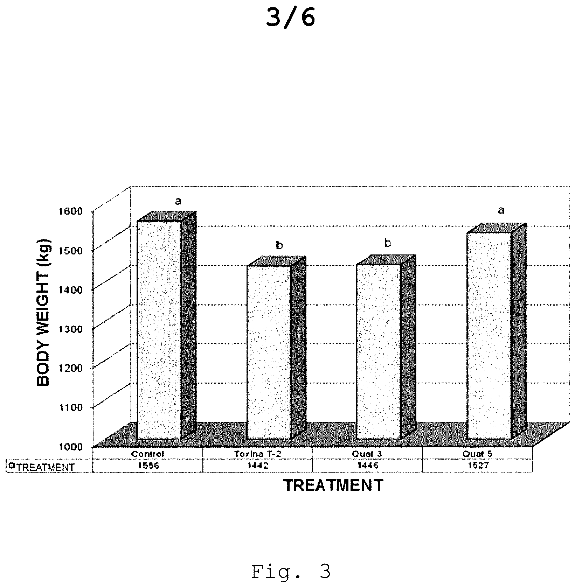

The body weight results following 28 days of testing are graphically represented in Figure 3 and Table 6, which also show weight gain and feed conversion.

**Table 6. Initial, final weights (28 days), Weight gain, Feed conversion.**

| **Treatments** | **Weight in g** | | **Weight gain** | **Feed conversion** |
|---|---|---|---|---|
| | Initial medians ± standard error | Final medians ± standard error | Medians ± standard error | Medians ± standard error |
| Negative control | 43 ± 0.46^{a} | 1556 ± 18.76^{a} | 1513 ± 18.87^{a} | 1.34 ± 0.017^{a} |
| Positive control T-2 toxin | 43 ± 0.43^{a} | 1442 ± 29.49^{b} | 1399 ± 29.46^{b} | 1.45 ± 0.035^{b} |
| Challenge QUAT 3 | 43 ± 0.42^{a} | 1446 ± 18.82^{b} | 1403 ± 18.81^{b} | 1.44 ± 0.020^{a} |
| Challenge QUAT 5 | 43 ± 0.42^{a} | 1527 ± 18.81^{a} | 1484 ± 18.75^{a} | 1.42 ± 0.018^{ab} |

| | | | | |
|---|---|---|---|---|
| Medians with different letters are statistically significant for p < 0.05. | | | | |

As it can be observed in Figure 3 as well as in Table 6, the effect of T2 toxin at levels of 1.8 ppm could indeed be observed in the animals, because statistical difference could be seen in both the final weight and the weight gain between the positive and negative control groups. It was also observed that the adsorbent with "QUAT 5" or "Q5" protected the chickens up to **74.6%** more than the control groups and statistically, there was no difference from the negative control group, hence the prototype indeed managed to reduce the T2 toxicity. To the contrary, it is important to note that the mycotoxin adsorbent with "QUAT 3" or "Q3" did not protect the chickens against the 1.8 ppm of T2 toxin.

We also observed that feed consumption was not affected despite the presence of oral lesions in the animals, even though reduction of feed consumption is normally a symptom of T-2 intoxication. These effects can be observed in Table 7, which also illustrates the effect of T-2 toxin in the xantophylls, which are pigments.

**Table 7. Feed consumption and xantophylls in oral treatment.**

| **Treatments** | **Kcantophylls (oral solution) Feed consumption mg/L** | |
|---|---|---|
| | Medians ± standard error | Medians ± standard error |
| Negative control | 2028 ± 20.5^{a} | 12.8 ± 0.47^{a} |
| Positive control T-2 toxin | 2035 ± 44.3^{a} | 8.92 ±0.70^{b} |
| Challenge QUAT 5 | 2101 ± 16.9^{a} | 12.7 ± 0.56^{a} |

| | | |
|---|---|---|
| Medians with different letters are statistically significant for p < 0.05. | | |

As we mentioned before, one of the typical symptoms of T-2 toxins are oral lesions in the chickens, which were indeed observed in the experiment. Figure 4 illustrates the oral lesions on the birds, produced by this toxin.

In order to obtain a numerical estimation of the degree of the lesions caused by T-2 toxin, the lesions were examined and assigned with the following numerical ratings: No lesions: 0. Minor lesions +:1. Moderate lesions ++: 2. Severe lesions +++: 3. Based on this classification system, Table 8 shows that despite the presence of lesions in the group given the mycotoxin adsorbent "QUAT 5" or "Q5", they were less severe than in the positive control group. The lesions in the negative control group were attributed to the type of meal feed. Based on this ranking, it can be concluded that the mycotoxin adsorbent with "QUAT 5" provided partial protection. However, these lesions did not significantly affect weight gain.

**Table 8. Oral lesions observed in chickens after consuming T2 toxin.**

| **Treatments** | **Chickens with oral lesions after 21 to 28 days** | | Numerical lesion score |
|---|---|---|---|
| | Affected chickens | Degree of lesion | |
| Negative control | 5/24 | 1 (+++) | 3 |
| Positive control T-2 toxin | 28/28 | 3 (+) | 63 |
| | | 15 (++) | |
| | | 10 (+++) | |
| Challenge QUAT 5 | 26/27 | 8 (+) | 46 |
| | | 16 (++) | |
| | | 2 (+++) | |

At the end of the experiment, the sacrificed chickens were evaluated based on various biological and histopathological factors in order to study the effects of T2 toxin and the efficacy of the prototype in reducing these effects. The tables containing the results, including measurements of organs such as livers and kidneys, hematological readings, vaccine response and histopathology of organs are presented below.

**Table 9.**

| **Treatments** | **Relative weight of liver (%)** | **Relative weight of kidney (%)** | **Relative weight of spleen (%)** |
|---|---|---|---|
| | Medians ± standard error | Medians ± standard error | Medians ± standard error |
| Negative control | 2.39 ± 0.068^{a} | 0.799 ± 0.018^{a} | 0.100 ± 0.006^{a} |
| Positive control T-2 toxin | 2.57 ±0.104^{a} | 0.809 ± 0.032^{a} | 0.094 ± 0.009^{a} |
| Challenge QUAT 5 | 2.53 ± 0.075^{a} | 0.846 ± 0.024^{a} | 0.098 ± 0.006^{a} |

| | | | |
|---|---|---|---|
| Medians with different letters are statistically significant for p < 0.05. | | | |

**Table 10.**

| **Treatments** | **Relative weight of thymus (%)** | **Relative weight of bursa of Fabricius (%)** |
|---|---|---|
| | Medians ± standard error | Medians ± standard error |
| Negative control | 0.318 ± 0.013^{a} | 0.254 ± 0.017^{a} |
| Positive control T-2 toxin | 0.282 ± 0.020^{a} | 0.248 ± 0.016^{a} |
| Challenge QUAT 5 | 0.328 ± 0.016^{a} | 0.237 ± 0.013^{a} |

| | | |
|---|---|---|
| Medians with different letters are statistically significant for p < 0.05. | | |

**Table 11.**

| **Treatments** | **White blood cells (Leukocytes) 10³/µL** | **Lymphocytes 10³/µL** | **Median cell count (monocytes, basophils and eosinophils) 10³/µL** | **Granulocytes (segmented neutrophils and band neutrophils) 10³/µL** |
|---|---|---|---|---|
| | Medians ± standard error | Medians ± standard error | Medians ± standard error | Medians ± standard error |
| Negative control | 83.5 ± 5.06^{a} | 72.1 ± 3.34^{a} | 10.0 ± 1.55^{a} | 1.4 ± 0.29^{a} |
| Positive control T-2 toxin | 65.10 4.07^{b} | 58.4 ± 3.32^{b} | 5.8 ± 0.76^{a} | 0.9 ± 0.17^{a} |
| Challenge QUAT 5 | 75.1 ± 4.00^{ab} | 66.2 ± 2.59^{ab} | 7.6 ± 1.28^{a} | 1.3 ± 0.42^{a} |

| | | | | |
|---|---|---|---|---|
| Medians with different letters are statistically significant for p < 0.05. | | | | |

**Table 12.**

| **Treatments** | **Red blood cells (erythrocytes) 10⁶/µL** | **Hemoglobin g/dL** | **Hematocrit %** | **Platelets 10³/µL** |
|---|---|---|---|---|
| | Medians ± standard error | Medians ± standard error | Medians ± standard error | Medians ± standard error |
| Negative control | 2.44 ± 0.06^{a} | 12.3 ± 0.37^{a} | 31.3 ± 0.83^{a} | 28 ± 1.92^{a} |
| Positive control T-2 toxin | 2.46 ±0.12^{a} | 12.1 ± 0.46^{a} | 31.8 ± 1.56^{a} | 31 ± 4.95^{a} |
| Challenge QUAT 5 | 2.34 ± 0.13^{a} | 12.4 ± 0.27^{a} | 30.4 ± 1.49^{a} | 29 ± 3.22^{a} |

| | | | | |
|---|---|---|---|---|
| Medians with different letters are statistically significant for p < 0.05. | | | | |

**Table 13.**

| **Treatments** | **Ash %** | **Phosphorous %** | **Calcium %** | **Manganesiu m mg/kg** | **Zinc mg/kg** |
|---|---|---|---|---|---|
| | Medians ± standard error | Medians ± standard error | Medians ± standard error | Medians ± standard error | Medians ± standard error |
| Negative control | 52.45 ± 0.85^{b} | 9.03 ± 0.15^{b} | 19.11 ± 0.31^{b} | 7.0 ± 0.17^{a} | 239 ± 4.87^{a} |
| Positive control T-2 toxin | 56.37 ±0.32^{a} | 9.72 ±0.07^{a} | 20.43 ± 0.19^{a} | 6.7 ± 0.21^{a} | 247 ± 4.37^{a} |
| Challenge QUAT 5 | 54.53 ± 0.34^{a} | 9.13 ± 0.09^{b} | 19.18 ± 0.15^{b} | 6.6 ± 0.22^{a} | 250 ± 4.95^{a} |

| | | | | | |
|---|---|---|---|---|---|
| Medians with different letters are statistically significant for p < 0.05. | | | | | |

**Table 14.**

| **Treatments** | **Inhibition of Newcastle hemagglutination (18 days after vaccination) Log₂** |
|---|---|
| | Medians ± standard error |
| Negative control | 9.207 ± 0.30^{ab} |
| Positive control T-2 toxin | 8.717 ± 0.29^{b} |
| Challenge QUAT 5 | 9.709 ± 0.29^{a} |

### Histopathology

### Negative control:

Proventriculus (12):
Lymphoid clusters observed in the glandular zone (1/12).
No significant changes (11/12).

Liver (12):
No significant changes (11/12).
Lymphocyte clusters observed around the portal cavities and between the sinusoids (1/12).
Morphological diagnosis: Minor multifocal lymphocytic hepatitis

Spleen, gizzard, thymus, tongue, kidney (12):
No significant changes (12/12).

Bursa of Fabricius (12):
No significant changes (11/12).
In one follicle, a necrosis center surrounded by giant cells (granuloma) is observed (1/12).
Morphological diagnosis: Minor localized bursitis granulomatosa. (Bacterial etiology).

COMMENTS: No mycotoxic lesions observed.

### Positive control:

Tongue (12): No significant changes (4/12).

Above the epithelium lining, dense plates are observed, composed of cellular remains combined with residual keratin and bacterial colonies. In the submucosa adjacent to said plates, lymphocyte and macrophage clusters can be found combined with necrotic cellular residue (8/12).
Morphological diagnosis: Estomatitis and erosive/necrotic glositis with presence of interlesional bacteria.

Larynx (12): No significant changes (4/12).
In the submucosa and around the glands, moderate or abundant inflammatory infiltration composed primarily of lymphocytes is observed (8/12).

Morphological diagnosis: Moderate to severe diffuse lymphocytic laryngitis.

Proventriculus (12): No significant changes (11/12).
Moderate hyperplasia of the associated lymphoid tissue is observed (1/12).

Liver (8): Around the portal cavities and in the parenchymal, multifocal lymphocyte clusters can be observed (8/12) along with areas of extramedullary hematopoiesis (1/12).
Morphological diagnosis: Moderate, multifocal lymphocytic colangiohepatitis.
Scant fat vacuoles can be observed in the hepatocyte cytoplasm (3/12).
Morphological diagnosis: Minor to diffuse moderate esteatosis.

Gizzard (12): Loss of continuity, ulcer activity observed, combined with abundant bacterial colonies and moderate heterophyllic infiltration (7/12).
Morphological diagnosis: Moderate multifocal ulcerative ventriculitis.
Scant erosions in the recovery zone can be observed (5/12).
Morphological diagnosis: Discrete, multifocal erosions.

Spleen, thymus (12):
No significant changes (12/12).

Kidney (12): Degeneration and necrosis of the epithelial cells of the tubules is observed (4/12). It is observed that some glomeruli resemble cells due to the proliferation of mesangium cells and the enlarging of the membrane caused by proliferation of endothelial cells. (4/12). Lymphocyte clusters are found in the interstitial space (4/12).

Morphological diagnosis:
Moderate diffuse proliferative membrane glomerulopathy with moderate multifocal interstitial lymphocytic nefritis.

### Challenge "QUAT 5" or "Q5":

Tongue (12): No significant changes (6/12).
Above the epithelium lining, dense plates composed of cellular remains mixed with keratin residue and bacterial colonies is observed. In the submucosa adjacent to said plates, lymphocyte and macrophage clusters mixed with necrotic cellular residue are observed (6/12).
Morphological diagnosis: Estomatitis and erosive/necrotic glositis with presence of intralesional bacteria.

Larynx (12): No significant changes (6/12).
In the submucosa and around the glands, moderate or abundant inflammatory infiltration is observed, mainly consisting of lymphocytes (6/12).
Morphological diagnosis: Moderate or severe diffuse lymphocytic laringitis.

Proventriculus (12): No significant changes (11/12).
Associated lymphoid cluster observed in the glandular area (1/12).

Liver (12): No significant changes (5/12).
Multifocal lymphocyte clusters are observed around the portal cavities and in the parenchymal (2/12).
Morphological diagnosis: Moderate, multifocal lymphocytic colangiohepatitis
Scant fat cells are observed in the cytoplasm, clear spaces observed in the interstitial areas (edema) (5/12).
Morphological diagnosis: Minor diffuse esteatosis.
Gizzard (12):
Loss of continuity, ulcer activity observed, combined with abundant bacterial colonies and moderate heterophyllic infiltration (4/12).
Morphological diagnosis: Moderate, multifocal ulcerative ventriculitis.
Scant erosions in the recovery zone are observed (8/12).
Morphological diagnosis: Discrete, multifocal erosions.

### Conclusion for Example 5

Example 5 demonstrated that 1.8 ppm of T2 toxin in the feed affected productive factors in the boiler chickens, as it had a significant impact on weight gain.

Based on the results presented, it can be concluded that the mycotoxin adsorbent with "QUAT 5" was efficient in protecting the chickens from the toxic effects of 1.8 ppm T2 toxin. On the contrary, the adsorbent with "QUAT 3" did not exhibit any protective effect.

Therefore, it can be concluded that the formulation of the mycotoxin adsorbent with "QUAT 5" is adequate for not only vomitoxin adsorbent but also for T2 toxin and generally, for trichothecenes.

In view of the foregoing, it is apparent that even though the specific embodiments of the invention were outlined herein for the purpose of illustration, several modifications thereof can be carried out without diverging from the nature and scope of the invention. Consequently, the invention is not subject to any limitation beyond what is listed in the claims below.

### EXAMPLE 6

### "Live" testing of a mycotoxin adsorbent of the invention designed to combat vomitoxin (or deoxinivalenol) on pigs

Another experiment was carried out on pigs to evaluate the efficacy of the mycotoxin adsorbent, an organically modified aluminum silicate with an alkyl phenol ethoxylate derivative that has the formula (Ia), which hereinafter we will refer to as: "QUAT 5". This is to verify the positive performance reported in Example 4.

### Analysis of the results of Example 6

24 recently weaned female piglets were used in this experiment, distributed in 4 groups, with 6 piglets each, each animal considered a replicate. Their diets were as reflected in Table 15. In this instance, a contamination of only 2500 ppb of vomitoxin was used with the objective of reaching values similar to the maximum recommended by the European Union (900 ppb).

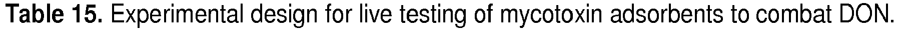

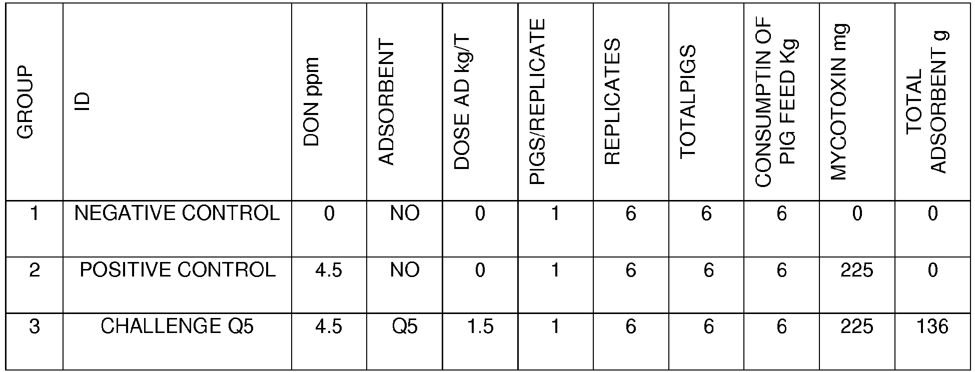

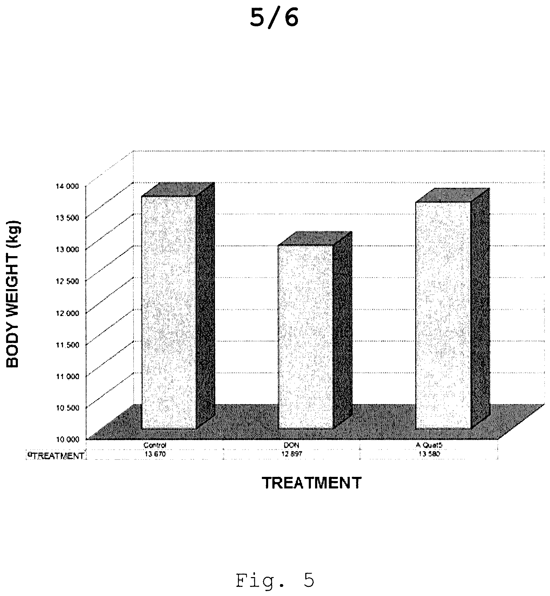

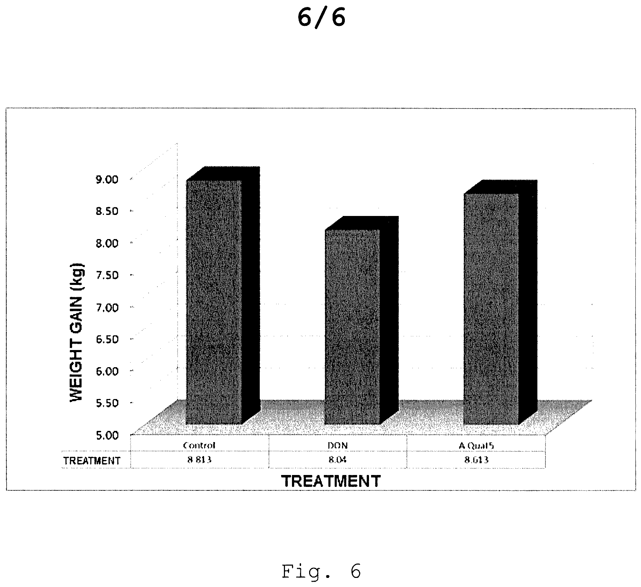

The body weight results following 23 days of testing are graphically represented in Figure 5 for the final weights and Figure 6 for the accumulated weight gain.

Figures 5 and 6 clearly show that the mycotoxin adsorbent "QUAT 5" provides protection against 2500 ppb of vomitoxin at 74.1%.

### Conclusion

The obtained results demonstrate that the mycotoxin adsorbent "QUAT 5," at a contamination of 2500 ppb of DON, provides a significant recovery of consumption and void the toxic effects of mycotoxins.

## Claims

1. A mycotoxin adsorbent comprising an aluminum silicate organically modified with an alkyl phenol ethoxylate derivative of formula (I): 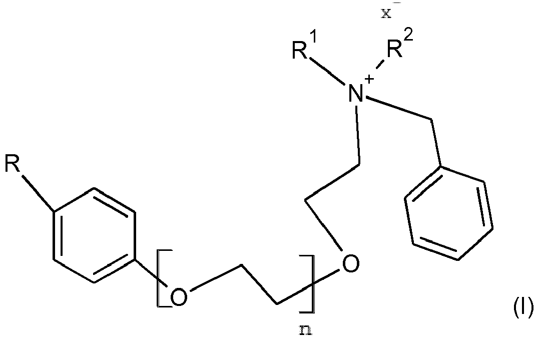 wherein R is n-nonyl, X is bromine, n = 9, R¹ is methyl and R² is methyl, and wherein the aluminium silicate has a cation exchange capacity of at least 20 milliequivalents per 100 g of material.

2. The mycotoxin adsorbent of claim 1, wherein the aluminum silicate is a tectosilicate, a phyllosilicate or a mixture of both.

3. The mycotoxin adsorbent of any of claims 1 to 2, wherein the aluminum silicate has a cation exchange capacity of 55 milliequivalents per 100 grams of material.

4. The mycotoxin adsorbent of any of claims 1 to 3, wherein the alkyl phenol ethoxylate derivative is present in a proportion of 25% to 120% of the cation exchange capacity of the aluminum silicate.

5. An additive for balanced animal feed comprising a mycotoxin adsorbent of any of claims 1 to 4.

6. A formulation of balanced animal feed that includes a mycotoxin adsorbent of any of claims 1 to 4.

7. A mycotoxin adsorbent of any of claims 1 to 4 for use in the treatment or prevention of one or more symptoms in the digestive tract associated with type A and/or B trichothecene intoxication; as additive for animal fodder, those symptoms being
vomiting associated with type A and/or B trichothecene intoxication;
diarrhea associated with type A and/or B trichothecene intoxication;
irritation in the digestive tract associated with type A and/or B trichothecene intoxication;
hemorrhaging in the digestive tract associated with type A and/or B trichothecene intoxication;
necrosis in the digestive tract associated with type A and/or B trichothecene intoxication.

8. A process for the preparation of a mycotoxin adsorbent of any of claims 1 to 4 comprising the following steps:
contacting an aluminum silicate with a cation exchange capacity of at least 20 milliequivalents per 100 grams of material with an alkyl phenol ethoxylate derivative of formula (I) in a proportion of 25% to 120% of the cation exchange capacity of the aluminum silicate, in an aqueous medium agitating it at a temperature between 15°C to 85° C for 0.25 to 3 hours;
separating the mycotoxin absorbent form the aqueous medium by filtration;
drying the filtered mycotoxin absorbent at a temperature between 40°C and 150°C; and
crush or grind with mesh between 100 and 325.

9. A process of claim 8, wherein the aluminum silicate has a cation exchange capacity of 55 milliequivalents per 100 grams of material.

## Patentansprüche

1. Mykotoxinadsorptionsmittel, umfassend ein Aluminiumsilikat, das organisch mit einem Alkylphenolethoxylatderivat der Formel (I) modifiziert ist: 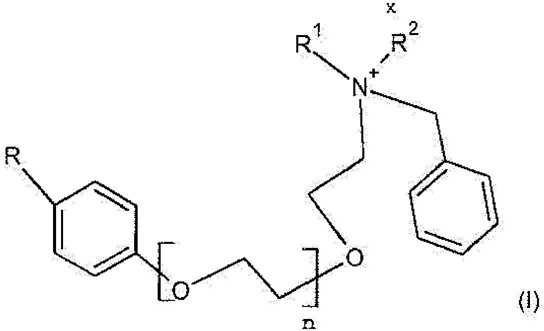 wobei R n-Nonyl ist, X Brom ist, n = 9, R¹ Methyl ist und R² Methyl ist und wobei das Aluminiumsilikat eine Kationenaustauschkapazität von mindestens 20 Milliäquivalenten pro 100 g Material aufweist.

2. Mykotoxinadsorptionsmittel nach Anspruch 1, wobei das Aluminiumsilikat ein Tektosilikat, ein Phyllosilikat oder eine Mischung von beiden ist.

3. Mykotoxinadsorptionsmittel nach einem der Ansprüche 1 bis 2, wobei das Aluminiumsilikat eine Kationenaustauschkapazität von 55 Milliäquivalenten pro 100 Gramm Material aufweist.

4. Mykotoxinadsorptionsmittel nach einem der Ansprüche 1 bis 3, wobei das Alkylphenolethoxylatderivat in einem Anteil von 25 % bis 120 % der Kationenaustauschkapazität des Aluminiumsilikats vorliegt.

5. Additiv für ausgewogenes Tierfutter, umfassend ein Mykotoxinadsorptionsmittel nach einem der Ansprüche 1 bis 4.

6. Formulierung eines ausgewogenen Tierfutters, die ein Mykotoxinadsorptionsmittel nach einem der Ansprüche 1 bis 4 enthält.

7. Mykotoxinadsorptionsmittel nach einem der Ansprüche 1 bis 4 zur Verwendung bei der Behandlung oder Vorbeugung eines oder mehrerer Symptome im Verdauungstrakt, die mit einer Trichothecen-Vergiftung vom Typ A und/oder B verbunden sind; als Additiv für Tierfutter, wobei diese Symptome sind
Erbrechen im Zusammenhang mit einer Trichothecen-Vergiftung vom Typ A und/oder B;
Durchfall im Zusammenhang mit einer Trichothecen-Vergiftung vom Typ A und/oder B;
Reizung im Verdauungstrakt im Zusammenhang mit einer Trichothecen-Vergiftung vom Typ A und/oder B;
Blutungen im Verdauungstrakt im Zusammenhang mit einer Trichothecen-Vergiftung vom Typ A und/oder B;
Nekrose im Verdauungstrakt im Zusammenhang mit einer Trichothecen-Vergiftung vom Typ A und/oder B.

8. Verfahren zur Herstellung eines Mykotoxinadsorptionsmittels nach einem der Ansprüche 1 bis 4, umfassend die folgenden Schritte:
Inkontaktbringen eines Aluminiumsilikats mit einer Kationenaustauschkapazität von mindestens 20 Milliäquivalenten pro 100 Gramm Material mit einem Alkylphenolethoxylatderivat der Formel (I) in einem Anteil von 25 % bis 120 % der Kationenaustauschkapazität des Aluminiumsilikats, in einem wässrigen Medium, das bei einer Temperatur zwischen 15 °C und 85 °C 0,25 bis 3 Stunden lang gerührt wird;
Abtrennen des Mykotoxinabsorptionsmittels von dem wässrigen Medium durch Filtration;
Trocknen des filtrierten Mykotoxinabsorptionsmittels bei einer Temperatur zwischen 40 °C und 150 °C; und
Zerdrücken oder Mahlen mit einer Maschenweite zwischen 100 und 325.

9. Verfahren nach Anspruch 8, wobei das Aluminiumsilikat eine Kationenaustauschkapazität von 55 Milliäquivalenten pro 100 Gramm Material aufweist.

## Revendications

1. Adsorbant de mycotoxines comprenant un silicate d'aluminium organiquement modifié avec un dérivé d'alkylphénol éthoxylate de formule (I) : 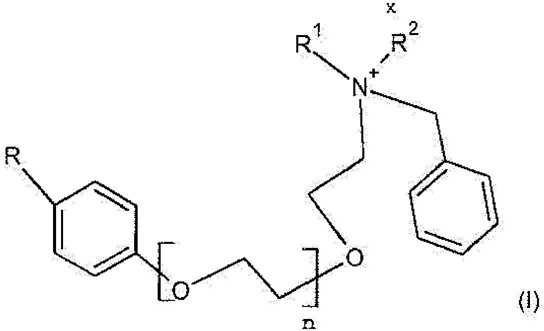 dans laquelle R représente un groupe n-nonyle, X représente un atome de brome, n = 9, R¹ représente un groupe méthyle et R² représente un groupe méthyle, et dans lequel le silicate d'aluminium a une capacité d'échange de cations d'au moins 20 milliéquivalents pour 100 g de substance.

2. Adsorbant de mycotoxines selon la revendication 1, dans lequel le silicate d'aluminium est un tectosilicate, un phyllosilicate ou un mélange des deux.

3. Adsorbant de mycotoxines selon l'une quelconque des revendications 1 à 2, dans lequel le silicate d'aluminium a une capacité d'échange de cations de 55 milliéquivalents pour 100 grammes de substance.

4. Adsorbant de mycotoxines selon l'une quelconque des revendications 1 à 3, dans lequel le dérivé alkylphénol éthoxylate est présent à raison de 25 % à 120 % de la capacité d'échange de cations du silicate d'aluminium.

5. Additif pour aliments équilibrés pour animaux comprenant un adsorbant de mycotoxines selon l'une quelconque des revendications 1 à 4.

6. Formulation d'aliments équilibrés pour animaux comprenant un adsorbant de mycotoxines selon l'une quelconque des revendications 1 à 4.

7. Adsorbant de mycotoxines selon l'une quelconque des revendications 1 à 4, destiné à être utilisé dans le traitement ou la prévention d'un ou plusieurs symptômes du tube digestif associés à une intoxication par les trichothécènes de type A et/ou B ; comme additif pour le fourrage animal, ces symptômes étant
des vomissements associés à une intoxication par les trichothécènes de type A et/ou B ;
une diarrhée associée à une intoxication par les trichothécènes de type A et/ou B ;
une irritation du tube digestif associée à une intoxication par les trichothécènes de type A et/ou B ;
des hémorragies dans le tube digestif associées à une intoxication par les trichothécènes de type A et/ou B ;
une nécrose du tube digestif associée à une intoxication par les trichothécènes de type A et/ou B.

8. Procédé de préparation d'un adsorbant de mycotoxines selon l'une quelconque des revendications 1 à 4, comprenant les étapes suivantes consistant à :
mettre en contact un silicate d'aluminium avec une capacité d'échange de cations d'au moins 20 milliéquivalents pour 100 grammes de substance avec un dérivé d'alkylphénol éthoxylate de formule (I) à raison de 25 % à 120 % de la capacité d'échange de cations du silicate d'aluminium, dans un milieu aqueux en l'agitant à une température comprise entre 15 °C et 85 °C pendant 0,25 à 3 heures ;
séparer l'absorbant de mycotoxines du milieu aqueux par filtration ;
sécher l'absorbant de mycotoxines filtré à une température comprise entre 40 °C et 150 °C ; et
écraser ou broyer avec un maillage compris entre 100 et 325.

9. Procédé selon la revendication 8, dans lequel le silicate d'aluminium a une capacité d'échange de cations de 55 milliéquivalents pour 100 grammes de substance.
